# EUROPEAN PATENT SPECIFICATION

(11) **EP 2 928 681 B1**
(45) Date of publication and mention of the grant of the patent: **09.06.2021**
(21) Application number: 12810044.3
(22) Date of filing: 07.12.2012
(51) Int. Cl.: B30B 11/08, B30B 11/34, A23G 4/04, A23G 3/34

(54) **COMPRESSED TABLETS**
GEPRESSTE TABLETTEN
COMPRIMÉS

(43) Date of publication of application: 14.10.2015
(73) Proprietor: Fertin Pharma A/S, 7100 Vejle (DK)
(72) Inventor: DALENTOFT, Tony, DK-8464 Galten (DK); SCHMIDT, Niels Ravn, DK-6092 Sønder Stenderup (DK)
(74) Representative: Patentgruppen A/S
(86) International application number: PCT/DK2012/050455
(87) International publication number: WO 2014/086358

(56) References cited:
- WO-A1-2004/004478
- WO-A1-2009/006892
- WO-A1-2009/007769
- WO-A1-2012/004133
- GB-A- 439 534
- GB-A- 743 222
- GB-A- 1 034 713
- US-A- 1 248 571
- US-A1- 2003 143 272
- US-A1- 2006 039 872
- US-A1- 2006 263 476
- US-A1- 2011 146 504

## Description

### FIELD

The present invention relates to the field of compressed tablets, such as compressed chewing gum tablets.

### BACKGROUND

Compressed tablets have tranditionally been manufactured on the basis of a powdered tablet base material under high pressure in a rotary tablet press. Due to the nature of a rotary tablet press and the high speed used in the manufacture of compressed tablets, it has previously been difficult to apply other materials than powdered tablet base in the manufacturing process of compressed tablets.

The document GB-A-1034713 discloses a method for producing compressed tablets with a core inserted between two layers of a granular material.

### SUMMARY OF THE INVENTION

Accordingly, there is provided a method according to the features and steps of claim

In a preferred embodiment of the invention, the object comprises liquid and a membrane as a barrier.

### DETAILED DESCRIPTION OF THE INVENTION

The terms "powdered portion of tablet base material" is intended to mean a portion of discrete particles of tablet base material.

The terms "base material" is intended to mean a material that forms the basis of the material, usually constituting a major part of the material.

The terms "first powdered portion of tablet base material" is intended to be understood as a first portion in a sequence of portions added.

The terms "second powdered portion of tablet base material" is intended to be understood as a second portion added after a first portion.

The terms "gum base", "gum base matrix" or "gum base portion" is intended to mean the mainly water insoluble and hydrophobic gum base ingredients that are mixed together before the bulk portion of the chewing gum composition is added.

The term "bulk portion" intends to mean the mainly water soluble and hydrophilic chewing gum ingredients that are mixed into the gum base matrix after the gum base matrix has been prepared.

By the phrase "chewing gum" is meant any chewing gum such as extruded chewing gum, centre-filled chewing gum, toffee-imitating chewing gum, compressed chewing gum, slabs or sticks.

By the phrase "granules of gum base" is meant granules consisting of gum base.

By the phrase "granules of chewing gum" is meant granules consisting of granulated chewing gum, wherein said chewing gum comprises gum base.

By the phrase "compressed chewing gum" is meant a chewing gum comprising granules or powder being exposed to a punching means in a tableting machine, pressing the granules or powder to a coherent mass of compressed material.

By the phrase "object" is meant a discrete or individual module, separate from the powdered portion of tablet base material. The "object" has an appearance of a visually distinct part of the tablet, usually, on the face of it, a solid material that may be handled by machinery without sticking, substantially without helping aids. The object preferably comprises a liquid or semi-liquid and a membrane. The size of the object will be larger than typical gum base granules, such as with a weight of more than 10mg.

By the phrase "membrane" is meant a barrier that substantially does not allow, or at least prevents to a substantial degree, flow of particles from one side of the membrane to the other side of the membrane.

By the phrase "texture" is meant a qualitative measure of the visco-elastic properties the chewing gum and of the overall mouth-feel experienced by the user during the chewing process. Thus the term "texture" encompasses measurable quantities such as hardness and elasticity as well as more subjective parameters related to the chew-feel experienced by a user.

The phrase "hydrophobic" is used to describe the ability of a substance to dissolve in or blend with apolar substances such as e.g. oils.

The phrase "hydrophilic" is used to describe the ability of a substance to dissolve in or blend with polar substances, such as e.g. water.

The term "sustained" or "extended" is herein intended to mean prolonged over time.

One of the benefits of the present invention is that there may be provided a better texture and taste of the final compressed tablet. Another benefit is that the new insight allows for inclusion of active ingredients in the centre of compresed tablets. This may be a benefit if the active ingredients would not be compatible with the powdered portion of tablet base material, or for instance if a higher amount of active ingredients is to be included in the compressed tablet. Another benefit is that ingredients may be more easily separated in the compressed tablets, for instance if ingredients may interact with the powdered tablet base material.

In some embodiments of the disclosure, at least 30% of the object in the central cavity is below the upper face of the first pressed material.

In some embodiments of the disclosure, at least 40% of the object in the central cavity is below the upper face of the first pressed material.

In some embodiments of the disclosure, at least 50% of the object in the central cavity is below the upper face of the first pressed material.

In some embodiments of the disclosure, at least 5% of the object in the central cavity is below the upper face of the first pressed material.

In some embodiments of the disclosure, at least 10% of the object in the central cavity is below the upper face of the first pressed material.

These embodiments may be an advantage in order to allow the object to be fixed during rotation in the rotary tablet press. Usually, the speed of a rotary tablet press is high and it may be a problem that the object would be forced away from the central part of the first powdered portion of tablet base material due to the centrifugal force in the tablet press.

In some embodiments of the disclosure, the object is made to fit into the central cavity.

Under some circumstances it may be an advantage that the object is designed to fit the cavity and that the contact area between the object and the pressed first tablet base material may substantially be maximized.

In some embodiments of the disclosure, the insertion of the object is performed by means of a separate rotary tablet press.

Typically, the process of manufacture is performed in a single rotary tablet press for cost reasons. But it may be a benefit under some circumstances to apply a separate rotary tablet press. This may be the case to allow for an easier and smoother insertion of the object into the tablet press. In some embodiments, the separate rotary tablet press runs in the same direction as the "main" tablet press, which may allow the object to be inserted in the die means from the separate rotary tablet press.

In some embodiments of the disclosure, the central cavity is formed by the upper punch during pressing of said first tablet base material.

Preferably, the upper punch is convex which gives the upper face of the pressed tablet base material a concave form. However, in order to have a more simple set-up of the rotary tablet machinery, it may be a benefit to use a punch which may be used to make a central cavity at the upper face of the pressed tablet base material.

In some embodiments of the disclosure, the central cavity is formed by a separate device after pressing of said first tablet base material.

While there are benefits by using a punch which allows obtaining a central cavity in the pressed first tablet base material, it may under some circumstances be a benefit to make the cavity in a separate process. This may be performed with yet another punch, designed to make the hole, or it may be done with an insertion device.

In some embodiments of the disclosure, the central cavity is formed during inserting the object at the upper face of the first pressed material.

In some embodiments of the disclosure, the central cavity is formed by the object during inserting the object at the upper face of the first pressed material.

Under some conditions, the object itself may be used to "penetrate" the pressed tablet base material and insert the object herein. This may in particular be useful, if the object is composed of a material that is harder to deform. In case the first powdered portion of tablet base material is only slightly pressed, the object may more easily be fixed in the powdered tablet base material.

In some embodiments of the disclosure, the upper face of the first pressed material is formed as a circle.

In some embodiments of the disclosure, the upper face of the first pressed material is formed as a circle and is concave.

In some embodiments of the disclosure, the central cavity is situated in the center of the upper face of the first pressed material.

In some embodiments of the disclosure, fixing the object in the central cavity of the first pressed material is further supported by a viscous material.

Considering that the force in the rotary tablet press is extensive, it may be a benefit to use a viscous material that may "glue" the object tighter to the first pressed material. This material may be polyol syrup.

In some embodiments of the disclosure, pressing said first tablet base material is performed at a force of 0.01 to 20 kN.

Accordingly, the force may be relatively low in some embodiments. The benefit of a low force is to allow the powdered portion of the first tablet base material to form a sharp line at the interface of the die.

Another benefit is that the object may be fixed to a higher degree in the central cavity of the first pressed tablet base material.

In some embodiments of the disclosure, the step of pressing said first tablet base material is performed at a force of 0.1 to 15 kN.

In some embodiments of the disclosure, the step of pressing said first tablet base material is performed at a force of 1 to 10 kN.

In some embodiments of the disclosure, the step of pressing said first tablet base material is performed at a force to allow for inserting the object at the upper face of the first pressed material without breaking the first pressed material.

In some embodiments of the disclosure, the second powdered portion of tablet base material is added into the punch die in an amount to fully cover the object.

In some embodiments of the disclosure, the weight ratio between the first and second powdered portion of tablet base material is 1:10 to 10:1.

In some embodiments of the disclosure, the weight ratio between the first and second powdered portion of tablet base material is 1:5 to 5:1.

In some embodiments of the disclosure, the weight ratio between the first and second powdered portion of tablet base material is 1:3 to 3:1.

In some embodiments of the disclosure, the weight ratio between the first and second powdered portion of tablet base material is 1:2 to 2:1.

In some embodiments of the disclosure, the weight ratio between the first and second powdered portion of tablet base material is 1:1.2 to 1.2:1.

In some embodiments of the invention, the first powdered portion of tablet base material comprises particles of polyol sweetener.

In some embodiments of the disclosure, the amount of polyol sweetener is above 20% by weight of the first powdered portion of tablet base material.

In some embodiments of the disclosure, the amount of polyol sweetener is above 40% by weight of the first powdered portion of tablet base material.

In some embodiments of the disclosure, the amount of polyol sweetener is above 60% by weight of the first powdered portion of tablet base material.

In some embodiments of the disclosure, the amount of polyol sweetener is above 80% by weight of the first powdered portion of tablet base material.

In some embodiments of the disclosure, the amount of polyol sweetener is above 95% by weight of the first powdered portion of tablet base material.

In some embodiments of the invention, the first powdered portion of tablet base material comprises granules of chewing gum.

In some embodiments of the disclosure, the first powdered portion of tablet base material consists of granules of chewing gum.

In some embodiments of the invention, the first powdered portion of tablet base material comprises granules of gum base.

In some embodiments of the invention, the amount of granules of gum base is in the range of 5 to 80% by weight of the first powdered portion of tablet base material.

In some embodiments of the invention, the amount of granules of gum base is in the range of 10 to 70% by weight of the first powdered portion of tablet base material. In some embodiments of the invention, the amount of granules of gum base is in the range of 20 to 60% by weight of the first powdered portion of tablet base material.

In some embodiments of the invention, the amount of granules of gum base is in the range of 30 to 50% by weight of the first powdered portion of tablet base material.

In some embodiments of the invention, the second powdered portion of tablet base material comprises particles of polyol sweetener.

In some embodiments of the disclosure, the amount of polyol sweetener is above 20% by weight of the second powdered portion of tablet base material.

In some embodiments of the disclosure, the amount of polyol sweetener is above 40% by weight of the second powdered portion of tablet base material.

In some embodiments of the disclosure, the amount of polyol sweetener is above 60% by weight of the second powdered portion of tablet base material.

In some embodiments of the disclosure, the amount of polyol sweetener is above 80% by weight of the second powdered portion of tablet base material.

In some embodiments of the disclosure, the amount of polyol sweetener is above 95% by weight of the second powdered portion of tablet base material.

In some embodiments of the invention, the second powdered portion of tablet base material comprises granules of chewing gum.

In some embodiments of the disclosure, the second powdered portion of tablet base material consists of granules of chewing gum.

In some embodiments of the invention, the second powdered portion of tablet base material comprises granules of gum base.

In some embodiments of the invention, the amount of granules of gum base is in the range of 5 to 80% by weight of the second powdered portion of tablet base material. In some embodiments of the invention, the amount of granules of gum base is in the range of 10 to 70% by weight of the second powdered portion of tablet base material. In some embodiments of the invention, the amount of granules of gum base is in the range of 20 to 60% by weight of the second powdered portion of tablet base material. In some embodiments of the invention, the amount of granules of gum base is in the range of 30 to 50% by weight of the second powdered portion of tablet base material. In some embodiments of the invention, the first powdered portion of tablet base material does not comprise gum base.

In some embodiments of the invention, the second powdered portion of tablet base material does not comprise gum base.

In some embodiments of the invention, the first powdered portion of tablet base material is different from the second powdered portion of tablet base material.

In some embodiments of the disclosure, the granules of chewing gum have a particle size ranging from 20 µm to 500 µm.

In some embodiments of the disclosure, the granules of gum base has a particle size ranging from 300 µm to 1300 µm.

In some embodiments of the disclosure, pressing of the first and second tablet base material is performed at a force of 16 to 60 kN.

In some embodiments of the disclosure, pressing of the first and second tablet base material is performed at a force of 20 to 50 kN.

In some embodiments of the disclosure, pressing of the first and second tablet base material is performed at a force of 30 to 40 kN.

In some embodiments of the invention, the object is situated in the cavity substantially in the centre of the tablet.

In some embodiments of the invention, the surface of the object is subjected to equal force per square milimeter during pressing said first and second tablet base material around the object.

In accordance with the invention, the weight ratio between the powdered tablet base material in the tablet and the object is 20:1 to 3:1.

In some embodiments of the invention, the weight ratio between the powdered tablet base material in the tablet and the object is 15:1 to 4:1.

In some embodiments of the disclosure, the weight ratio between the powdered tablet base material in the tablet and the object is 10:1 to 5:1.

In some embodiments of the invention, the object is substantially ball-shaped.

In some embodiments of the disclosure, the object is a capsule.

In some embodiments of the invention, the object comprises a liquid and a membrane as a barrier.

In some embodiments of the invention, the object comprises a semi-liquid and a membrane as a barrier.

In some embodiments of the disclosure, the object comprises a high viscous liquid and a membrane as a barrier.

In some embodiments of the disclosure, the object comprises a low viscous liquid and a membrane as a barrier.

In some embodiments of the disclosure, the object comprises syrup and a membrane as a barrier.

In some embodiments of the disclosure, the object comprises polyol syrup and a membrane as a barrier.

In some embodiments of the disclosure, the object comprises oily liquid and a membrane as a barrier.

In some embodiments of the disclosure, the object comprises a jelly material and a membrane as a barrier.

In some embodiments of the invention, the object comprises gelatine and a membrane as a barrier.

In some embodiments of the disclosure, the object comprises a solid material.

In some embodiments of the disclosure, the object comprises a solid material and enzymes to allow the solid material to become a liquid.

In some embodiments of the disclosure, the object comprises a gum base.

In some embodiments of the invention, the object comprises an active ingredient.

In some embodiments of the invention, the object comprises a nutraceutical ingredient.

In some embodiments of the invention, the object comprises a pharmaceutical active ingredient.

In some embodiments of the invention, the object comprises a functional ingredient.

In some embodiments of the disclosure, the object has a weight of more than 10mg, such as more than 50mg.

In another embodiment of the disclosure, the compressed tablet is a compressed mint tablet. In this embodiment, the first portion of tablet base material may consist of tablet base material with an amount of polyol and the second portion of tablet base material may consist of tablet base material with an amount of polyol.

In some embodiments of the disclosure, the gum base content is in the range of 10 to 50% by weight of the tablet.

In some embodiments of the disclosure, the gum base content is in the range of 15 to 45% by weight of the tablet.

In some embodiments of the disclosure, the gum base content is in the range of 20 to 40% by weight of the tablet.

In embodiments of the present disclosure, the gum base comprises
- elastomer in the range of 5-40% by weight of the gum base,
- natural resin in the range of 8-45% by weight of the gum base, and
- synthetic resin in the range of 5-95% by weight of the gum base.

In embodiments of the present disclosure, the tablet comprises natural resins in an amount of 0.1 to 40%, preferably 1 to 30%, such as 3 to 25% or 5 to 20%, by weight of the tablet.

In embodiments of the present disclosure, the tablet comprises natural resins in an amount of at least 13% by weight of the tablet.

In embodiments of the present disclosure, the tablet comprises synthetic resins in an amount of 0.1 to 40%, preferably 1 to 30%, such as 3 to 25% or 5 to 20%, by weight of the tablet.

In embodiments of the present disclosure, the tablet comprises elastomer in an amount of at least 2% by weight of the chewing gum formulation, preferably at least 4% by weight of the tablet.

In embodiments of the present disclosure, the tablet comprises elastomer in an amount of less than 35% by weight of the chewing gum formulation, preferably less than about 25% by weight of the chewing gum formulation such as less than 20%, 15% or 10% by weight of the tablet.

In embodiments of the present disclosure, the tablet comprises one or more flavoring agents selected from the group consisting of essential oils, essences, extracts, powders, acids, coconut, coffee, chocolate, vanilla, grape fruit, orange, lime, menthol, liquorice, caramel aroma, honey aroma, peanut, walnut, cashew, hazelnut, almonds, pineapple, strawberry, raspberry, apple, pear, peach, apricot, blackberry, cherry, pineapple, plum essence, clove oil, bay oil, anise, thyme, cedar leaf oil, nutmeg, cinnamon, peppermint, wintergreen, spearmint, eucalyptus, mint, or any combination thereof.

In embodiments of the present disclosure, the tablet comprises humectants, such as propylene glycol or glycerol.

In embodiments of the present disclosure, the tablet comprises enhancers such as cocoa solids, licorice, tobacco extracts, and sugars.

In embodiments of the present disclosure, the tablet is provided with a coating.

In embodiments of the present disclosure, the tablet has a weight in the range of 0.1 to 10 grams, preferably in the range of 0.5 to 4 grams.

In embodiments of the present disclosure, the tablet comprises filler in an amount of 0.1 to 50% by weight of the chewing gum.

According to an advantageous embodiment of the disclosure, the tablet may comprise filler.

In embodiments of the present disclosure, the tablet comprises filler in an amount of 0.1 to 50% by weight of the tablet, wherein the filler is hydrophobic and wherein at least 90% of the filler is contained in the tablet throughout the chewing of a user during a chewing period of at least 10 minutes.

In some embodiments of the disclosure, a buffer is added, the buffer being selected from the group consisting of a tris buffers, amino acid buffers, carbonate, including monocarbonate, bicarbonate or sesquicarbonate, glycerinate, phosphate, glycerophosphate, acetate, glyconate or citrate of an alkali metal, such as potassium and sodium, e.g. trisodium and tripotassium citrate, or ammonium, and mixtures thereof.

When buffer is used, a preferred buffer is sodium bicarbonate. In some embodiments buffer is not part of the chewing gum formulation. In some other embodiments, buffer is part of the chewing gum formulation.

In some embodiments of the disclosure, the amount of buffer is 0.5 to 10% by weight of the tablet.

In some embodiments of the disclosure, the buffer is selected from the group consisting of a carbonate, including monocarbonate, bicarbonate or sesquicarbonate, glycerinate, phosphate, glycerophosphate, acetate, glyconate or citrate of an alkali metal, such as potassium and sodium, e.g. trisodium and tripotassium citrate, or ammonium, tris buffer, amino acids, and mixtures thereof.

However, in a presently preferred embodiment an alkaline buffer is preferred, such as sodium carbonate.

Elastomers provide the rubbery, cohesive nature to the gum, which varies depending on this ingredient's chemical structure and how it may be compounded with other ingredients. Elastomers suitable for use in the gum base and gum of the present invention may include natural or synthetic types.

Elastomer plasticizers vary the firmness of the gum base. Their specificity on elastomer inter-molecular chain breaking (plasticizing) along with their varying softening points cause varying degrees of finished gum firmness and compatibility when used in base. This may be important when one wants to provide more elastomeric chain exposure to the alkane chains of the waxes.

The elastomers (rubbers) employed in the gum base may vary depending upon various factors such as the type of gum base desired, the texture of gum formulation desired and the other components used in the formulation to make the final chewing gum product. The elastomer may be any water-insoluble polymer known in the art, and includes those gum polymers utilized for chewing gums and bubble gums. Illustrative examples of suitable polymers in gum bases include both natural and synthetic elastomers. For example, those polymers which are suitable in gum base formulations include, without limitation, natural substances (of vegetable origin) such as chicle gum, natural rubber, crown gum, nispero, rosidinha, jelutong, perillo, niger gutta, tunu, balata, guttapercha, lechi capsi, sorva, gutta kay, and the like, and mixtures thereof. Examples of synthetic elastomers include, without limitation, styrene-butadiene copolymers (SBR), polyisobutylene, isobutylene-isoprene copolymers, polyethylene, polyvinyl acetate and the like, and mixtures thereof.

Natural resins may be used and may be natural rosin esters, often referred to as ester gums including as examples glycerol esters of partially hydrogenated rosins, glycerol esters of polymerised rosins, glycerol esters of partially dimerized rosins, glycerol esters of tally oil rosins, pentaerythritol esters of partially hydrogenated rosins, methyl esters of rosins, partially hydrogenated methyl esters of rosins, pentaerythritol esters of rosins, synthetic resins such as terpene resins derived from alpha-pinene, beta-pinene, and/or d-limonene, and natural terpene resins.

In an embodiment of the disclosure, the resin comprises terpene resins, e.g. derived from alpha-pinene, beta-pinene, and/or d-limonene, natural terpene resins, glycerol esters of gum rosins, tall oil rosins, wood rosins or other derivatives thereof such as glycerol esters of partially hydrogenated rosins, glycerol esters of polymerized rosins, glycerol esters of partially dimerised rosins, pentaerythritol esters of partially hydrogenated rosins, methyl esters of rosins, partially hydrogenated methyl esters of rosins or pentaerythritol esters of rosins and combinations thereof.

In an embodiment of the disclosure, the powdered tablet base materials are selected from the group consisting of bulk sweeteners, flavors, dry-binders, tabletting aids, anti-caking agents, emulsifiers, antioxidants, enhancers, absorption enhancers, buffers, high intensity sweeteners, softeners, colors, or any combination thereof.

In an embodiment of the disclosure, the powdered tablet base material comprises sweeteners, such as bulk sweeteners, sugar sweeteners, sugar substitute sweeteners, artificial sweeteners, high-intensity sweeteners, or any combination thereof. Suitable bulk sweeteners include both sugar and non-sugar sweetening components.

Bulk sweeteners typically constitute from about 5 to about 95% by weight of the tablet, more typically about 20 to about 80% by weight such as 30 to 70% or 30 to 60% by weight of the tablet.

Useful sugar sweeteners are saccharide-containing components commonly known in the tablet art including, but not limited to, sucrose, dextrose, maltose, dextrins, trehalose, D-tagatose, dried invert sugar, fructose, levulose, galactose, corn syrup solids, and the like, alone or in combination.

Sorbitol can be used as a non-sugar sweetener. Other useful non-sugar sweeteners include, but are not limited to, other sugar alcohols such as mannitol, xylitol, hydrogenated starch hydrolysates, maltitol, isomalt, erythritol, lactitol and the like, alone or in combination.

High intensity artificial sweetening agents can also be used alone or in combination with the above sweeteners. Preferred high intensity sweeteners include, but are not limited to sucralose, aspartame, salts of acesulfame, alitame, saccharin and its salts, cyclamic acid and its salts, glycyrrhizin, dihydrochalcones, thaumatin, monellin, stevioside (natural intensity sweetener) and the like, alone or in combination. In order to provide longer lasting sweetness and flavor perception, it may be desirable to encapsulate or otherwise control the release of at least a portion of the artificial sweeteners. Techniques such as wet granulation, wax granulation, spray drying, spray chilling, fluid bed coating, conservation, encapsulation in yeast cells and fiber extrusion may be used to achieve desired release characteristics. Encapsulation of sweetening agents can also be provided using another tablet component such as a resinous compound.

Usage level of the artificial sweetener will vary considerably and will depend on factors such as potency of the sweetener, rate of release, desired sweetness of the product, level and type of flavor used and cost considerations. Thus, the active level of artificial sweetener may vary from about 0.001 to about 8% by weight (preferably from about 0.02 to about 8% by weight). When carriers used for encapsulation are included, the usage level of the encapsulated sweetener will be proportionately higher. Combinations of sugar and/or non-sugar sweeteners may be used in the chewing gum formulation.

A tablet according to the disclosure may, if desired, include one or more fillers/texturisers including as examples, magnesium and calcium carbonate, sodium sulphate, ground limestone, silicate compounds such as magnesium and aluminum silicate, kaolin and clay, aluminum oxide, silicium oxide, talc, titanium oxide, mono-, di- and tri-calcium phosphates, cellulose polymers, such as wood, and combinations thereof.

A number of tablet base materials well known within the art may be applied within the scope of the present invention. Such components comprise but are not limited to waxes, fats, softeners, fillers, flavors, anti-oxidants, emulsifiers, colouring agents, binding agents and acidulants

In an embodiment of the disclosure, the tablet is provided with an outer coating.

In an embodiment of the disclosure, said outer coating is selected from the group consisting of hard coating, soft coating and edible film-coating or any combination thereof

In an embodiment of the disclosure, said tablet comprises one or more encapsulation delivery systems.

In one embodiment of the disclosure, the flavor may be used as taste masking in the tablet comprising active ingredients, which by themselves have undesired taste or which alter the taste of the formulation.

The gum base granules according to the disclosure are preferably made by means of extrusion and under-water pelletizing.

The size of gum base granules according to the present disclosure are controlled by several factors such as opening sizes, the gum composition, gum temperature and pressure drop, if a die plate is used in the extruder. Due to an interaction between the pressurized gum composition, temperature and friction in the openings of the die device, the average diameter of the produced granules are normally larger than the diameters of the openings in the die device. The relation between the diameters of the openings in the die device and the average diameters of granules produced from a specific gum composition may be determined by the skilled person on basis of routine experiments.

It is also possible to produce granules with different average diameters by making granules with one diameter, and subsequently mix the granules with different average diameters in desired proportions.

Although the openings of the die device may have cross-sections of any desired shape, e.g. circular, oval, square etc., it is preferred that the die device comprises openings with substantially circular cross-section and diameters in the range of 0.1 to 1.3 mm. A first set of openings can e.g. have a first diameter in the range of 0.07 to 0.7 mm, preferably in the range of 0.15 to 0.6 mm, and suitably in the range of 0.2 to 0.5 mm. A second set of openings can have a second diameter larger than said first diameter. The second diameter is conveniently in the range of 0.4 to 1.3 mm, preferably in the range of 0.7 to 1.2 mm.

Preferably the chewing gum granulating system further comprises a drying device. Powder sweetener or talk may be added to the granules in a final drying step. The drying device can be a conventional centrifugal dryer or another suitable dryer e.g. a fluid bed dryer. The drying device can, for example, include a mixer. The powder sweetener is preferably sorbitol, which is mixed to the dried or partially dried granules. Minor amounts of residual moisture on the surface of the granules, e.g. 2% Wt. based on the total weight of the granules, may contribute to the adherence of the sorbitol powder to the surface of the granules. It is possible to use a conventional anti-agglomerating agent as e.g. talcum, but sorbitol powder can function as an anti-agglomerating agent, and at the same time serves as sweetener. Although sorbitol is found to be most suitable, other bulk sweeteners based on polyols may also be suitable, e.g. mannitol, xylitol, hexa-resorcinol, maltitol, isomaltol, erythriol, and lactitol.

In a preferred embodiment the chewing gum granulating system according to the disclosure further comprises one or more sieves adapted for removing granules with an average diameter such as above 1.3 mm. The removal of larger granules improves a subsequent tabletting process.

According to the disclosure it is preferred that at least the extruder and/or the die device comprises means for controlling the temperature of the gum composition. The means for controlling temperature can be cooling or heating devices, and may serve to facilitate the flow of gum composition through the extruder and the die device. In an embodiment the extruder comprises delivering means for delivering sweetener and/or flavour to the gum composition in the extruder.

During extrusion of the gum composition the differential pressure between the gum composition in the extruder and the gum composition in the liquid filled chamber, i.e. over the die device is suitably above 10 bar, preferably above 18 bar, such as in the range of 25 to 90 bar. The temperature of the gum composition in the extruder is preferably in the range of 40 to 125° C., suitably in the range 50 to 115° C. The temperature of the die device is preferably in the range of 60 to 250° C, suitably in the range 80 to 180° C. The temperature of the liquid in the liquid filled chamber is conveniently in the range of 8 to 40° C. The optimum for the pressures and temperatures in the method according to the invention may, however, may be determined by the skilled person as a matter of routine. The optimum values for specific gum compositions, varies of course, depending on the composition.

The quick cooling in the air filled or water-filled chamber may act to preserve possible fragile ingredients in the gum composition so that their qualities are better kept intact and conveyed into the granules included in the final gum product. This improved quality of the gum composition in the granules improves the general composition of the chewing gum product.

Granule fractions of different average weights may be produced with two different setups, each producing a batch of granules of a particular average weight, followed by a blending of the fractions. It is also possible to design a die means with die openings of at least two different sizes to simultaneously obtain granules with different average diameter. Thus it is possible to obtain granules having different weights. More than two different average weights may be obtained, depending on the design of the die means in use. It is for instance possible to obtain granules with three, four or more different average weights although two different weights are preferred.

The granules may be cut in a very large liquid-filled chamber, in which the granules are also cooled, but preferably the cooling is combined with transfer of the granules away from the chamber. This can be done e.g. by cooling the cut granules in water during transfer from the liquid filled chamber to a de-watering device. The transfer time from cutting to de-watering can be less than 6 s. The advantage of this is that water-soluble ingredients in the gum composition are not unnecessarily washed out of the granules. Optionally, the total time of contact between granules and cooling water can be further limited to less than 4 s.

It is preferred that the gum composition fed to the extruder is a gum base, and that it at least includes one or more flavouring agents when extruded through the die means. The flavours within the granules cause a prolonged release of taste during mastication.

The powdered tablet base material according to the disclosure may for example comprise so-called primary particles or aggregated primary particles, also referred to as granules. When these are compressed, bonds are established between the particles or granules, thereby conferring a certain mechanical strength to the compressed tablet.

It should be noted that the above-introduced terms: powder, primary particles and granules may be somewhat misleading in the sense that the difference between primary particles and granules may very often be looked upon differently depending on the background of the user. Some may for instance regard a sweetener, such as sorbitol, as a primary particle in spite of the fact that sorbitol due to the typically preprocessing performed on sorbitol when delivered to the customer should rather be regarded as some sort of granule. The definition adopted in the description of this invention is that granules refer to macro-particles comprising more or less preprocessed primary particles.

When pressure is applied to the powder raw material, the bulk volume is reduced and the amount of air is decreased. During this process energy is consumed. As the particles come into closer proximity to each other during the volume reduction process, bonds may be established between the particles or granules. The formation of bonds is associated with a reduction in the energy of the system as energy is released.

Volume reduction takes place by various mechanisms and different types of bonds may be established between the particles or granules depending on the pressure applied and the properties of the particles or granules.

The first thing that happens when a powder is compressed is that the particles are rearranged under low compaction pressures to form a closer packing structure. Particles with a regular shape appear to undergo rearrangement more easily than those of irregular shape. As the pressure increases, further rearrangement is prevented and subsequent volume reduction is obtained by plastic and elastic deformation and/or fragmentation of the tablet particles. Brittle particles are likely to undergo fragmentation, i.e. breakage of the original particles into smaller units. Plastic deformation is an irreversible process resulting in a permanent change of particle shape, whereas the particles resume their original shape after elastic deformation. Evidently, both plastic and elastic deformation may occur, when compressing a chewing gum tablet.

Several studies of the bond types in compressed tablets have been made over the years, typically in the context of pharmaceuticals and several techniques of obtaining compressed tablets on the basis of available powders has been provided. Such studies have been quite focused one what happens when the volume reduction is performed and how may the end-product be optimized for the given purpose. Several refinements with respect to compressed tablets has for instance been made in the addition of for example binders in the tablet raw materials for the purpose of obtaining a sufficient strength to the final compressed tablet while maintaining acceptable properties, e.g. with respect to release.

Over the years, especially the pharmaceutical industry has gradually introduced chewing gum as a mean for obtaining release of active ingredients in the oral cavity. Traditionally, the compression technique has been preferred by the pharmaceutical industry for the manufacturing of chewing gum. As mentioned above, a problem related to the compression technique is that the nature of chewing gum granules is quite different to that of pure pharmaceutical conventional tablet powder. A further, and even more significant problem is that the required texture is basically completely different from that of a tablet intended for completely dissolving within the mouth of the user. Hence, this compression technique has been regarded as inferior with respect to the basic texture properties of therewith obtained chewing gum.

Over the last few years, however, the technique has rapidly improved especially with respect to development of gum base granulates intended for compression. Examples of such gum base granulate are described in the PCT/DK02/00461and PCT/DK02/00462.

Chewing gum base formulations may comprise one or more elastomeric compounds of synthetic origin selected from polyisobutylene, isobutylene-isoprene copolymer, styrene-butadiene copolymers, polyvinyl acetate, polyisoprene, polyethylene and vinyl acetate-vinyl burate copolymer, and typically one or more resinous compounds which may be of synthetic or natural origin, fillers, softening compounds and minor amounts of miscellaneous ingredients such as antioxidants and colorants, etc.

The composition of chewing gum base formulations, which are admixed with chewing gum ingredients as defined below, can vary substantially depending on the particular product to be prepared and on the desired masticatory and other sensory characteristics of the final product. However, typical ranges (weight%) of the above gum base components are: 5 to 50% by weight elastomeric compounds, 5 to 55% by weight elastomer plasticizers, 0 to 50% by weight filler/texturiser, 5 to 35% by weight softener and 0 to 1% by weight of miscellaneous ingredients such as antioxidants, colorants, etc.

Gum base granulates may be manufactured according to conventional methods or e.g. those described in the PCT/DK02/00461 and PCT/DK02/00462.

In the present context, tablet base material may include bulk sweeteners, high intensity sweeteners, flavoring agents, softeners, emulsifiers, coloring agents, binding agents, acidulants, fillers, antioxidants and other components such as pharmaceutically or biologically active substances that confer desired properties to the finished tablet.

Suitable bulk sweeteners include e.g. both sugar and non-sugar components. Bulk sweeteners typically constitute from about 5 to 95% by weight of the tablet, more typically about 20 to 80% by weight such as 30 to 60% by weight of the tablet. Useful sugar sweeteners are saccharide-containing components commonly known in the tablet art including, but not limited to, sucrose, dextrose, maltose, dextrins, trehalose, D-tagatose, dried invert sugar, fructose, levulose, galactose, corn syrup solids, and the like, alone or in combination.

Sorbitol can be used as a non-sugar sweetener. Other useful non-sugar sweeteners include, but are not limited to, other sugar alcohols such as mannitol, xylitol, hydrogenated starch hydrolysates, maltitol, isomalt, erythritol, lactitol and the like, alone or in combination.

High-intensity artificial sweetening agents are used alone or in combination with the above sweeteners. Preferred high-intensity sweeteners include, but are not limited to sucralose, aspartame, salts of acesulfame, alitame, saccharin and its salts, neotame, cyclamic acid and its salts, glycyrrhizin, dihydrochalcones, thaumatin, monellin, stevioside and the like, alone or in combination. In order to provide longer lasting sweetness and flavor perception, it may be desirable to encapsulate or otherwise control the release of at least a portion of the artificial sweetener. Likewise, encapsulation may be applied for the purpose of stabilizing the ingredients. Techniques such as wet granulation, wax granulation, spray drying, spray chilling, fluid bed coating, coascervation, encapsulation in yeast cells and fiber extrusion may be used to achieve the desired release characteristics. Encapsulation of sweetening agents can also be provided e.g. using another tablet component, such as a resinous compound, as the encapsulation agent.

Usage level of the artificial sweetener will vary considerably depending e.g. on factors such as potency of the sweetener, rate of release, desired sweetness of the product, level and type of flavor used and cost considerations. Thus, the active level of artificial sweetener may vary from about 0.02 to 8% by weight. When carriers used for encapsulation are included, the usage level of the encapsulated sweetener will be proportionally higher. Combinations of sugar and/or non-sugar sweeteners can be used in the chewing gum formulation processed in accordance with the invention. Additionally, the softener may also provide additional sweetness such as with aqueous sugar or alditol solutions.

If a low calorie gum is desired, a low calorie bulking agent can be used. Examples of low calorie bulking agents include polydextrose, Raftilose, Raftilin, Inuline, fructooligosaccharides (NutraFlora®, palatinose oligosaccharided; guar gum hydrolysates (e.g. Sun Fiber®) or indigestible dextrins (e.g. Fibersol®). However, other low calorie-bulking agents can be used.

Further tablet base material ingredients, which may be included in the tablet mixture processed in the present process, include surfactants and/or solubilisers, especially when pharmaceutically, cosmetically or biologically active ingredients are present. As examples of types of surfactants to be used as solubilisers in a chewing gum composition, according to the invention reference is made to H.P. Fiedler, Lexikon der Hilfstoffe für Pharmacie, Kosmetik und Angrenzende Gebiete, pages 63-64 (1981) and the lists of approved food emulsifiers of the individual countries. Anionic, cationic, amphoteric or non-ionic solubilisers can be used. Suitable solubilisers include lecithins, polyoxyethylene stearate, polyoxyethylene sorbitan fatty acid esters, fatty acid salts, mono and diacetyl tartaric acid esters of mono and diglycerides of edible fatty acids, citric acid esters of mono and diglycerides of edible fatty acids, saccharose esters of fatty acids, polyglycerol esters of fatty acids, polyglycerol esters of interesterified castor oil acid (E476), sodium stearoyllatylate, sodium lauryl sulfate and sorbitan esters of fatty acids and polyoxyethylated hydrogenated castor oil (e.g. the product sold under the trade name CREMOPHOR), block copolymers of ethylene oxide and propylene oxide (e.g. products sold under trade names PLURONIC and POLOXAMER), polyoxyethylene fatty alcohol ethers, polyoxyethylene sorbitan fatty acid esters, sorbitan esters of fatty acids and polyoxyethylene steraric acid esters.

Particularly suitable solubilisers are polyoxyethylene stearates, such as for instance polyoxyethylene(8)stearate and polyoxyethylene(40)stearate, the polyoxyethylene sorbitan fatty acid esters sold under the trade name TWEEN, for instance TWEEN 20 (monolaurate), TWEEN 80 (monooleate), TWEEN 40 (monopalmitate), TWEEN 60 (monostearate) or TWEEN 65 (tristearate), mono and diacetyl tartaric acid esters of mono and diglycerides of edible fatty acids, citric acid esters of mono and diglycerides of edible fatty acids, sodium stearoyllactylate, sodium laurylsulfate, polyoxyethylated hydrogenated castor oil, blockcopolymers of ethylene oxide and propyleneoxide and polyoxyethylene fatty alcohol ether. The solubiliser may either be a single compound or a combination of several compounds. The expression "solubiliser" is used in the present text to describe both possibilities; the solubiliser used must be suitable for use in food and/or medicine.

In the presence of an active ingredient the chewing gum may preferably also comprise a carrier known in the art.

One significant advantage of the present process is that the temperature throughout the entire operation can be kept at a relatively low level such as it will be described in the following. This is an advantageous feature with regard to preserving the aroma of added flavoring components, which may be prone to deterioration and/evaporation at higher temperatures. Aroma agents and flavoring agents which are useful in a chewing gum produced by the present process are e.g. natural and synthetic flavorings (including natural flavorings) in the form of freeze-dried natural vegetable components, essential oils, essences, extracts, powders, including acids and other substances capable of affecting the taste profile. Examples of liquid and powdered flavorings include coconut, coffee, chocolate, vanilla, grape fruit, orange, lime, menthol, liquorice, caramel aroma, honey aroma, peanut, walnut, cashew, hazelnut, almonds, pineapple, strawberry, raspberry, tropical fruits, cherries, cinnamon, peppermint, wintergreen, spearmint, eucalyptus, and mint, fruit essence such as from apple, pear, peach, strawberry, apricot, raspberry, cherry, pineapple, and plum essence. The essential oils include peppermint, spearmint, menthol, eucalyptus, clove oil, bay oil, anise, thyme, cedar leaf oil, nutmeg, and oils of the fruits mentioned above.

In one preferred embodiment, the flavor is one or more natural flavoring agent(s) which is/are freeze-dried, preferably in the form of a powder, slices or pieces of combinations thereof. The particle size of such agent may be less than 3 mm, such as less than 2 mm, more preferred less than 1 mm, and calculated as the longest dimension of the particle. The natural flavoring agent may also be in a form where the particle size is from about 3 µm to 2 mm, such as from 4 µm to 1 mm. Preferred natural flavoring agents include seeds from a fruit e.g. from strawberry, blackberry and raspberry.

Various synthetic flavors, such as mixed fruit flavor may also be used. As indicated above, the aroma agent may be used in quantities smaller than those conventionally used. The aroma agents and/or flavors may be used in an amount from 0.01 to about 30% by weight of the final product depending on the desired intensity of the aroma and/or flavor used. Preferably, the content of aroma/flavor is in the range of from 0.2 to 3% by weight of the total composition.

According to the disclosure, encapsulated flavors or active ingredient,s may be added to the final blend prior to compression.

Different methods of encapsulating flavors or active ingredients, which may both refer to flavors or active ingredients mixed into the gum base and flavors or active ingredients compressed into the chewing gum may e.g. include Spray drying, Spray cooling, Film coating, Coascervation, Double emulsion method (Extrusion technology) or Prilling.

Materials to be used for the above-mentioned encapsulation methods may e.g. include Gelatine, Wheat protein, Soya protein, Sodium caseinate, Caseine, Gum arabic, Mod. starch, Hydrolyzed starches (maltodextrines), Alginates, Pectin, Carregeenan, Xanthan gum, Locus bean gum, Chitosan, Bees wax, Candelilla wax, Carnauba wax, Hydrogenated vegetable oils, Zein and/or Sucrose.

The object of the present invention may as an example include Gelatine, Wheat protein, Soya protein, Sodium caseinate, Caseine, Gum arabic, Mod. starch, Hydrolyzed starches (maltodextrines), Alginates, Pectin, Carregeenan, Xanthan gum, Locus bean gum, Chitosan, Bees wax, Candelilla wax, Carnauba wax, Hydrogenated vegetable oils, Zein and/or Sucrose.

Active ingredients may be added to the tablet base material or be enclosed in the object.

Preferably, an active ingredient is enclosed in the object, such as nutraceuticals, pharmaceuticals or functional ingredients.

Preferably, these ingredients should be added subsequent to any significant heating or mixing. In other words, the active ingredients should preferably be added immediately prior to the compression of the final tablet.

Referring to the process, the adding of active ingredients may be cautiously blended with pre-mixed gum base granulates and further desired ingredients, immediately prior to the final compression of the tablet.

In one embodiment the tablet according to the invention comprises a pharmaceutically, cosmetically or biologically active substance. Examples of such active substances, a comprehensive list of which is found e.g. in WO 00/25598, include drugs, dietary supplements, antiseptic agents, pH adjusting agents, anti-smoking agents and substances for the care or treatment of the oral cavity and the teeth such as hydrogen peroxide and compounds capable of releasing urea during chewing. Examples of useful active substances in the form of antiseptics include salts and derivatives of guanidine and biguanidine (for instance chlorhexidine diacetate) and the following types of substances with limited water-solubility: quaternary ammonium compounds (e.g. ceramine, chloroxylenol, crystal violet, chloramine), aldehydes (e.g. paraformaldehyde), derivatives of dequaline, polynoxyline, phenols (e.g. thymol, p-chlorophenol, cresol), hexachlorophene, salicylic anilide compounds, triclosan, halogenes (iodine, iodophores, chloroamine, dichlorocyanuric acid salts), alcohols (3,4 dichlorobenzyl alcohol, benzyl alcohol, phenoxyethanol, phenylethanol), cf. also Martindale, The Extra Pharmacopoeia, 28th edition, pages 547-578; metal salts, complexes and compounds with limited water-solubility, such as aluminum salts, (for instance aluminum potassium sulphate AlK(SO₄)₂, 12H₂O) and salts, complexes and compounds of boron, barium, strontium, iron, calcium, zinc, (zinc acetate, zinc chloride, zinc gluconate), copper (copper chloride, copper sulphate), lead, silver, magnesium, sodium, potassium, lithium, molybdenum, vanadium should be included; other compositions for the care of mouth and teeth: for instance; salts, complexes and compounds containing fluorine (such as sodium fluoride, sodium monofluorophosphate, aminofluorides, stannous fluoride), phosphates, carbonates and selenium. Further active substances can be found in J. Dent. Res. Vol. 28 No. 2, pages 160-171,1949.

Examples of active substances in the form of agents adjusting the pH in the oral cavity include: acids, such as adipic acid, succinic acid, fumaric acid, or salts thereof or salts of citric acid, tartaric acid, malic acid, acetic acid, lactic acid, phosphoric acid and glutaric acid and acceptable bases, such as carbonates, hydrogen carbonates, phosphates, sulphates or oxides of sodium, potassium, ammonium, magnesium or calcium, especially magnesium and calcium.

Active ingredients may comprise the below mentioned compounds or derivates thereof but are not limited thereto: Acetaminophen, Acetylsalicylic acid, Buprenorphine, Bromhexin, Celcoxib, Codeine, Diphenhydramin, Diclofenac, Etoricoxib, Ibuprofen, Indometacin, Ketoprofen, Lumiracoxib, Morphine, Naproxen, Oxycodon, Parecoxib, Piroxicam, Pseudoefedrin, Rofecoxib, Tenoxicam, Tramadol, Valdecoxib, Calciumcarbonat, Magaldrate, Disulfiram, Bupropion, Nicotine, Azithromycin, Clarithromycin, Clotrimazole, Erythromycin, Tetracycline, Granisetron, Ondansetron, Prometazin, Tropisetron, Brompheniramine, Ceterizin, leco-Ceterizin, Chlorcyclizine, Chlorpheniramin, Chlorpheniramin, Difenhydramine, Doxylamine, Fenofenadin, Guaifenesin, Loratidin, des-Loratidin, Phenyltoloxamine, Promethazin, Pyridamine, Terfenadin, Troxerutin, Methyldopa, Methylphenidate, Benzalcon. Chloride, Benzeth. Chloride, Cetylpyrid. Chloride, Chlorhexidine, Ecabet-sodium, Haloperidol, Allopurinol, Colchinine, Theophylline, Propanolol, Prednisolone, Prednisone, Fluoride, Urea, Actot, Glibenclamide, Glipizide, Metformin, Miglitol, Repaglinide, Rosiglitazone, Apomorfin, Cialis, Sildenafil, Vardenafil, Diphenoxylate, Simethicone, Cimetidine, Famotidine, Ranitidine, Ratinidine, cetrizin, Loratadine, Aspirin, Benzocaine, Dextrometorphan, Phenylpropanolamine, Pseudoephedrine, Cisapride, Domperidone, Metoclopramide, Acyclovir, Dioctylsulfosucc., Phenolphtalein, Almotriptan, Eletriptan, Ergotamine, Migea, Naratriptan, Rizatriptan, Sumatriptan, Zolmitriptan, Aluminum salts, Calcium salts, Ferro salts, Ag-salts, Zinc-salts, Amphotericin B, Chlorhexidine, Miconazole, Triamcinolonacetonid, Melatonine, Phenobarbitol, Caffeine, Benzodiazepiner, Hydroxyzine, Meprobamate, Phenothiazine, Buclizine, Brometazine, Cinnarizine, Cyclizine, Difenhydramine, Dimenhydrinate, Buflomedil, Amphetamine, Caffeine, Ephedrine, Orlistat, Phenylephedrine, Phenylpropanolamin, Pseudoephedrine, Sibutramin, Ketoconazole, Nitroglycerin, Nystatin, Progesterone, Testosterone, Vitamin B 12, Vitamin C, Vitamin A, Vitamin D, Vitamin E, Pilocarpin, Aluminumaminoacetat, Cimetidine, Esomeprazole, Famotidine, Lansoprazole, Magnesiumoxide, Nizatide and or Ratinidine.

The invention is suitable for increased or accelerated release of active agents selected among the group of dietary supplements, oral and dental compositions, antiseptic agents, pH adjusting agents, anti-smoking agents, sweeteners, flavorings, aroma agents or drugs. Some of those will be described below.

The active agents to be used in connection with the present invention may be any substance desired to be released from the tablet. The active agents, for which a controlled and/or accelerated rate of release is desired, are primarily substances with a limited water-solubility, typically below 10 g/100 ml inclusive of substances which are totally water-insoluble. Examples are medicines, dietary supplements, oral compositions, anti-smoking agents, highly potent sweeteners, pH adjusting agents, flavorings etc.

Other active ingredients are, for instance, paracetamol, benzocaine, cinnarizine, menthol, carvone, caffeine, chlorhexidine-di-acetate, cyclizine hydrochloride, 1,8-cineol, nandrolone, miconazole, mystatine, sodium fluoride, nicotine, cetylpyridinium chloride, other quaternary ammonium compounds, vitamin E, vitamin A, vitamin D, glibenclamide or derivatives thereof, progesterone, acetylsalicylic acid, dimenhydrinate, cyclizine, metronidazole, sodium hydrogen carbonate, the active components from ginkgo, the active components from propolis, the active components from ginseng, methadone, oil of peppermint, salicylamide, hydrocortisone or astemizole.

Examples of active agents in the form of dietary supplements are for instance salts and compounds having the nutritive effect of vitamin B2 (riboflavin), B12, folinic acid, folic acid, niacine, biotine, poorly soluble glycerophosphates, amino acids, the vitamins A, D, E and K, minerals in the form of salts, complexes and compounds containing calcium, phosphorus, magnesium, iron, zinc, copper, iodine, manganese, chromium, selenium, molybdenum, potassium, sodium or cobalt.

Furthermore, reference is made to lists of nutritionists accepted by the authorities in different countries such as for instance US code of Federal Regulations, Title 21, Section 182.5013.182 5997 and 182.8013-182.8997.

Examples of active agents in the form of compounds for the care or treatment of the oral cavity and the teeth are for instance bound hydrogen peroxide and compounds capable of releasing urea during chewing.

Examples of active agents in the form of antiseptics are for instance salts and compounds of guanidine and biguanidine (for instance chlorhexidine diacetate) and the following types of substances with limited water-solubility: quaternary ammonium compounds (for instance ceramine, chloroxylenol, crystal violet, chloramine), aldehydes (for instance paraformaldehyde), compounds of dequaline, polynoxyline, phenols (for instance thymol, para chlorophenol, cresol) hexachlorophene, salicylic anilide compounds, triclosan, halogenes (iodine, iodophores, chloroamine, dichlorocyanuric acid salts), alcohols (3,4 dichlorobenzyl alcohol, benzyl alcohol, phenoxyethanol, phenylethanol), cf. furthermore Martindale, The Extra Pharmacopoeia, 28th edition, pages 547-578; metal salts, complexes and compounds with limited water-solubility, such as aluminum salts, (for instance aluminum potassium sulphate AlK(SO₄)₂,12H₂O) and furthermore salts, complexes and compounds of boron, barium, strontium, iron, calcium, zinc, (zinc acetate, zinc chloride, zinc gluconate), copper (copper chloride, copper sulfate), lead, silver, magnesium, sodium, potassium, lithium, molybdenum, vanadium should be included; other compositions for the care of mouth and teeth: for instance; salts, complexes and compounds containing fluorine (such as sodium fluoride, sodiummonofluorophosphate, amino fluorides, stannous fluoride), phosphates, carbonates and selenium.

Cf. furthermore J. Dent.Res. Vol. 28 No. 2, pages 160-171, 1949, wherein a wide range of tested compounds is mentioned.

Examples of active agents in the form of agents adjusting the pH in the oral cavity include for instance: acceptable acids, such as adipic acid, succinic acid, fumaric acid, or salts thereof or salts of citric acid, tartaric acid, malic acid, acetic acid, lactic acid, phosphoric acid and glutaric acid and acceptable bases, such as carbonates, hydrogen carbonates, phosphates, sulfates or oxides of sodium, potassium, ammonium, magnesium or calcium, especially magnesium and calcium.
Examples of active agents in the form of anti-smoking agents include for instance: nicotine, tobacco powder or silver salts, for instance silver acetate, silver carbonate and silver nitrate.

In a further embodiment, the sucrose fatty acid esters may also be utilized for increased release of sweeteners including for instance the so-called highly potent sweeteners, such as for instance saccharin, cyclamate, aspartame, thaumatin, dihydrocalcones, stevioside, glycyrrhizin or salts or compounds thereof. For increased released of sweetener, the sucrose fatty acids preferable have a content of palmitate of at least 40% such as at least 50%.

Further examples of active agents are medicines of any type.

Examples of active agents in the form of medicines include caffeine, salicylic acid, salicyl amide and related substances (acetylsalicylic acid, choline salicylate, magnesium salicylate, sodium salicylate), paracetamol, salts of pentazocine (pentazocine hydrochloride and pentazocinelactate), buprenorphine hydrochloride, codeine hydrochloride and codeine phosphate, morphine and morphine salts (hydrochloride, sulfate, tartrate), methadone hydrochloride, ketobemidone and salts of ketobemidone (hydrochloride), beta-blockers, (propranolol), calcium antagonists, verapamil hydrochloride, nifedinpine as well as suitable substances and salts thereof mentioned in Pharm. Int., Nov.85, pages 267-271, Barney H. Hunter and Robert L. Talbert, nitroglycerine, erythrityl tetranitrate, strychnine and salts thereof, lidocaine, tetracaine hydrochloride, etorphine hydrochloride, atropine, insulin, enzymes (for instance papain, trypsin, amyloglucosidase, glucoseoxidase, streptokinase, streptodornase, dextranase, alpha amylase), polypeptides (oxytocin, gonadorelin, (LH.RH), desmopressin acetate (DDAVP), isoxsuprine hydrochloride, ergotamine compounds, chloroquine (phosphate, sulfate), isosorbide, demoxytocin, heparin. Other active ingredients include beta-lupeol, Letigen®, Sildenafil citrate and derivatives thereof.

Dental products include Carbamide, CPP Caseine Phospho Peptide; Chlorhexidine, Chlorhexidine di acetate, Chlorhexidine Chloride, Chlorhexidine di gluconate, Hexetedine, Strontium chloride, Potassium Chloride, Sodium bicarbonate, Sodium carbonate, Fluor containing ingredients, Fluorides, Sodium fluoride, Aluminum fluoride.

Ammonium fluoride, Calcium fluoride, Stannous fluoride, Other fluor containing ingredients Ammonium fluorosilicate, Potassium fluorosilicate, Sodium fluorosilicate, Ammonium monofluorphosphate, Calcium monofluorphosphate, Potassium monofluorphosphate, Sodium monofluorphosphate, Octadecentyl Ammonium fluoride, Stearyl Trihydroxyethyl Propylenediamine Dihydrofluoride, Vitamins include A, B1, B2, B6, B12, Folinic acid, Folic acid, niacin, Pantothenic acid, biotine, C, D, E, K. Minerals include Calcium, phosphor, magnesium, iron, Zinc, Cupper, Iod, Mangan, Crom, Selene, Molybden. Other active ingredients include:
Q10®, enzymes. Natural drugs including Ginkgo Biloba, ginger, and fish oil.
The invention also relates to use of migraine drugs such as Serotonin antagonists: Sumatriptan, Zolmitriptan, Naratriptan, Rizatriptan, Eletriptan; nausea drugs such as Cyclizin, Cinnarizin, Dimenhydramin, Difenhydrinat; hay fever drugs such as Cetrizin, Loratidin, pain relief drugs such as Buprenorfin, Tramadol, oral disease drugs such as Miconazol, Amphotericin B, Triamcinolonaceton; and the drugs Cisaprid, Domperidon, Metoclopramid. In a preferred embodiment the disclosure relates to the release of Nicotine and its salts.

Above mentioned active ingredients and/or flavors may be pre-mixed into the gum base or of course added to the non-or low CG incorporated layer.

When the gum base granules comprises pre-mixed active ingredients, a controlled release of active ingredients may be obtained by means of at least a double active ingredients buffer. The first buffer comprising active ingredients blended into the final mix immediately prior to compression and the second buffer comprising active ingredients blended into the gum base prior to the blending of gum base and gum base ingredients.

In accordance with the disclosure, the chewing gum element comprises about 0 to about 75% by weight of an outer coating applied onto the chewing gum center. In the present context, a suitable outer coating is any coating that results in extended storage stability of the compressed chewing gum products as defined above, relative to a chewing gum of the same composition that is not coated. Thus, suitable coating types include hard coatings, film coatings and soft coatings of any composition including those currently used in coating of chewing gum, pharmaceutical products and confectioneries.

According to a preferred embodiment of the disclosure, film coating is applied to the compressed chewing gum tablet.

One presently preferred outer coating type is a hard coating, which term is used in the conventional meaning of that term including sugar coatings and sugar-free (or sugarless) coatings and combinations thereof. The object of hard coating is to obtain a sweet, crunchy layer which is appreciated by the consumer and to protect the gum centers for various reasons as. In a typical process of providing the chewing gum centers with a protective sugar coating the gum centers are successively treated in suitable coating equipment with aqueous solutions of crystallisable sugar such as sucrose or dextrose, which, depending on the stage of coating reached, may contain other functional ingredients, e.g. fillers, colors, etc. In the present context, the sugar coating may contain further functional or active compounds including flavor compounds, pharmaceutically active compounds and/or polymer degrading substances.

In the production of chewing gum it may, however, be preferred to replace the cariogenic sugar compounds in the coating by other, preferably crystallisable, sweetening compounds that do not have a cariogenic effect. In the art such coatings are generally referred to as sugarless or sugar-free coatings. Presently preferred non-cariogenic hard coating substances include polyols, e.g. sorbitol, maltitol, mannitol, xylitol, erythritol, lactitol, isomalt and tagatose which are obtained by industrial methods by hydrogenation of D-glucose, maltose, fructose or levulose, xylose, erythrose, lactose, isomaltulose and D-galactose, respectively.

In a typical hard coating process, as it will be described in details in the following, syrup containing crystallisable sugar and/or polyol is applied onto the gum centers and the water it contains is evaporated off by blowing with warm, dry air. This cycle must be repeated several times, typically 10 to 80 times, in order to reach the swelling required. The term "swelling" refers to the increase in weight of the products, as considered at the end of the coating operation by comparison with the beginning, and in relation to the final weight of the coated products. In accordance with the present invention, the coating layer constitutes for example about 0 to 75% by weight of the finished chewing gum element, such as about 10 to 60% by weight, including about 15 to 50% by weight.

In further useful embodiments, the membrane of the object may be based on a thin polymer-based coating applied to the object. The thickness of such a membrane is usually between 20 and 100 µm.

In the present context, suitable membrane materials include edible cellulose derivatives such as cellulose ethers including methylcellulose (MC), hydroxy ethyl cellulose (HEC), hydroxypropyl cellulose (HPC) and hydroxypropyl methylcellulose (HPMC). Other useful membrane agents are acrylic polymers and copolymers, e.g. methylacrylate aminoester copolymer or mixtures of cellulose derivatives and acrylic polymers. Such polymers may include methylacrylate ester copolymers, ethylcellulose (EC) and enteric polymers designed to resist the acidic stomach environment, yet dissolve readily in the duodenum. The latter group of polymers includes: cellulose acetate phthalate (CAP), polyvinyl acetate phthalate (PVAP), shellac, metacrylic acid copolymers, cellulose acetate trimellitate (CAT) and HPMC.

It will be appreciated that the membrane according to the method of the present invention may comprise any combination of the above film-coating polymers.

The gum base formulations applicable within the scope of the invention as defined in the appended claims comprise a synthetic elastomer selected from polyisobutylene. e.g. having a gas pressure chromatography (GPC) average molecular weight in the range of about 10,000 to 1,000,000 including the range of 50,000 to 80,000, isobutylene-isoprene copolymer (butyl elastomer), styrene-butadiene copolymers e.g. having styrene-butadiene ratios of about 1:3 to 3:1, polyvinyl acetate (PVA), e.g. having a GPC average molecular weight in the range of 2,000 to 90,000 such as the range of 3,000 to 80,000 including the range of 30,000 to 50,000, where the higher molecular weight polyvinyl acetates are typically used in bubble gum base, polyisoprene, polyethylene, vinyl acetate-vinyl laurate copolymer e.g. having a vinyl laurate content of about 5 to 50% by weight such as 10 to 45% by weight of the copolymer, and combinations hereof.
It is common in the industry to combine in a gum base a synthetic elastomer having a high molecular weight and a low molecular weight elastomer. Presently preferred combinations of synthetic elastomers include, but are not limited to, polyisobutylene and styrene-butadiene, polyisobutylene and polyisoprene, polyisobutylene and isobutylene-isoprene copolymer (butyl rubber) and a combination of polyisobutylene, styrene-butadiene copolymer and isobutylene isoprene copolymer, and all of the above individual synthetic polymers in admixture with polyvinyl acetate, vinyl acetate-vinyl laurate copolymers, respectively and mixtures thereof. Particularly interesting elastomeric or resinous polymer compounds which advantageously can be used in a process according to the invention include polymers which, in contrast to currently used elastomers and resins, can be degraded physically, chemically or enzymatically in the environment after use of the chewing gum, thereby giving rise to less environmental pollution than chewing gums based on non-degradable polymers, as the used degradable chewing gum remnants will eventually disintegrate and/or can be removed more readily by physical or chemical means from the site where it has been dumped.

In accordance with the disclosure, the chewing gum base components, which are used herein, may include one or more resinous compounds contributing to obtain the desired masticatory properties and acting as plasticizers for the elastomers of the gum base composition. In the present context, useful elastomer plasticizers include, but are not limited to, natural rosin esters, often referred to as ester gums including as examples glycerol esters of partially hydrogenated rosins, glycerol esters of polymerised rosins, glycerol esters of partially dimerised rosins, glycerol esters of tally oil rosins, pentaerythritol esters of partially hydrogenated rosins, methyl esters of rosins, partially hydrogenated methyl esters of rosins and pentaerythritol esters of rosins. Other useful resinous compounds include synthetic resins such as terpene resins derived from alpha-pinene, beta-pinene, and/or d-limonene, natural terpene resins; and any suitable combinations of the foregoing. The choice of elastomer plasticizers will vary depending on the specific application, and on the type of elastomer(s) being used.

The following non-limiting examples illustrate different variations of the present invention as defined in the claims.

### EXAMPLES

### Example 1

### Preparation of gum base

The composition of a gum base is presented in Table 1.

**Table 1 - Gum base composition. Amounts are given in wt-% of the gum base. GB=Gum Base.**

| | GB std. |
|---|---|
| Elastomer | 16.0 |
| Resins | 44.5 |
| Filler | 15.0 |
| Plasticizers | 24.4 |
| Antioxidant | 0.1 |

The preparation of gum base in this example is carried out by first adding a high-molecular weight elastomer, synthetic resin and filler to a heated (about 120°C) and running z-blade mixer. After about twenty minutes of mixing, natural resin is added to the running mixer and mixing is continued for about five minutes followed by addition of further natural resin. After about five minutes of continued mixing, some plasticizer and further elastomer are added to the running mixer, and mixing is continued for about five minutes before addition of further plasticizer and antioxidant to the running mixer. Mixing is continued for about half an hour to one hour, and the final gum base mass is emptied from the mixer into coated or lined pans, extruded or cast into any desirable shape. Those skilled in the art will recognize that many variations of the above-described procedure may be followed.

### Example 2

### Preparation of chewing gum

In the present example, the gum base standard from example 1 GB std. was made into chewing gum CG std. with the composition as described in Table 2.

**Table 2 - Amounts are given in % by weight of the chewing gum formulation. CG = Chewing Gum**

| | CG std. |
|---|---|
| GB std. | 42 |
| Bulk Sweetener Sorbitol | 57.3 |
| Intense sweeteners | 0.7 |

A conventional mechanical mixing procedure is used. The gum base is added to a mixing kettle provided with mixing means like e.g. horizontally placed Z-shaped arms. The kettle had been preheated to a temperature of up to approximately 50°C, and the other ingredients are added according to a specified time schedule. Obviously, the amount of ingredients used may be varied within the scope of the present invention as defined in the claims.

The chewing gum formulation may optionally be coated by means of hard coating. The coating may e.g. be applied according to conventional coating methods. The pieces evaluated are without coating.

### Example 3

### Preparation of gum base by continuous extrusion

The composition of gum bases are presented in Table 3.

**Table 3 - Gum base composition. Amounts are given in wt-% of the gum base. GB=Gum Base.**

| | GB103 | GB104 | GB105 | GB106 |
|---|---|---|---|---|
| Elastomer | 15 | 10 | 15 | 12 |
| Gum resins | 20 | 25 | 25 | 20 |
| Synthetic resins | 15 | 15 | 10 | 18 |
| Filler | 25 | 25 | 25 | 25 |
| Plasticizers | 15 | 15 | 15 | 15 |
| Emulsifier | 10 | 10 | 10 | 10 |
| Antioxidant | 0.1 | 0.1 | 0.1 | 0.1 |

A gum base composition in the form of pellets was fed directly to an extruder in a first opening.

The gum base pellets were fed individually to the extruder (Leistrits ZSE/BL 360 kw 104, available from GALA GmbH, Germany). The resulting composition was extruded to a granulator comprising a die plate and a water-filled chamber (granulator A5 PAC 6, GALA GmbH, Germany) connected to a water system comprising a water supply for the granulator and centrifugal dryer (TWS 20, available from GALA GmbH, Germany).

The individual gum base compositions (GB103-GB106) of Table 3 were fed to the extruder with a feed rate of 250 kg/h and an extruder screw speed of 200 rpm. The gum base compositions were made in separate productions. The temperature in the composition at the feed end of the extruder was 100°C and the temperature of the composition at the outlet of the extruder was 109°C. The composition was delivered by the extruder device to the inlet side of a die plate at a pressure of 36 bar. The composition was extruded through the die plate having a temperature of 200°C and 1100 holes of a diameter of 0.3 mm. In the granulator chamber the extruded composition was cut to granules by a cutter with 13 blades mounted in star shape on a central axle rotating with a cutter speed of 2800 rpm. The granules were cooled and transported to the centrifugal dryer in water with a temperature of 17°C and a flow rate of 22 m³/h. The average cooling and transport time in water was approx. 90 seconds. The individual granules had an average weight of 0.002 g.

In an alternative embodiment, the individual gum base compositions (GB103-GB106) of Table 3 were fed to the extruder with a feed rate of 250 kg/h and delivered to the inlet side of the die plate at a temperature of 110°C and a pressure of 52 bar. The gum base compositions were made in separate productions. The composition was extruded through a die plate having a temperature of 119°C and 24 holes of a diameter of 0.3 mm. In the granulator chamber the extruded composition was cut to granules by a cutter with 13 blades on a central axle rotating with a cutter speed of 2800 rpm. The granules were cooled and transported to the centrifugal dryer in water with a temperature of 18°C and a flow rate of 23 m³/h. The average cooling and transport time in water was approx. 90 seconds.

In a further alternative embodiment the individual gum base composition (GB103-GB 106) of Table 3 was fed to the extruder with a feed rate of 200 kg/h and delivered to the inlet side of the die plate at a temperature of 111°C and a pressure of 72 bar. The gum base compositions were made in separate productions. The composition was extruded through a die plate having a temperature of 200°C and 700 holes of a diameter of 0.2 mm. In the granulator chamber the extruded composition was cut to granules by a cutter with 13 blades on a central axle rotating with a cutter speed of 2800 rpm. The granules were cooled and transported to the centrifugal dryer in water with a temperature of 19°C and a flow rate of 23 m³/h. The average cooling and transport time in water was approx. 90 seconds.

In a further alternative embodiment the gum base composition (GB103-GB106) of Table 3 was fed to the extruder with a feed rate of 250 kg/h and delivered to the inlet side of the die plate at a temperature of 109°C and a pressure of 71 bar. The gum base compositions were made in separate productions. The composition was extruded through a die plate having a temperature of 177°C and 1500 holes of a diameter of 0.36 mm. In the granulator chamber the extruded composition was cut to granules by a cutter with 13 blades on a central axle rotating with a cutter speed of 2700 rpm. The granules were cooled and transported to the centrifugal dryer in water with a temperature of 19°C and a flow rate of 22 m³/h. The average cooling and transport time in water was approx. 90 seconds.

### Example 4

### Preparation of compressed chewing gum

The composition of compressed chewing gum is presented in Table 4.

**Table 4 - Compressed chewing gum composition. Amounts are given in wt-% of the chewing gum formulation.**

| | CG103 | CG104 | CG105 | CG106 |
|---|---|---|---|---|
| GB103 | 32 | | | |
| GB104 | | 32 | | |
| GB105 | | | 32 | |
| GB106 | | | | 32 |
| Sorbitol | 21.5 | 21.5 | 21.5 | 21.5 |
| Acesulfame K | 0.1 | 0.1 | 0.1 | 0.1 |
| Aspartame | 0.2 | 0.2 | 0.2 | 0.2 |
| Buffer | 1.5 | 1.5 | 1.5 | 1.5 |
| Flavor | 1.5 | 1.5 | 1.5 | 1.5 |
| Xylitol | 43.2 | 43.2 | 43.2 | 43.2 |

The gum base granules obtained in Example 3 (GB103-GB106) were individually mixed in a standard mixer with tablet base material in the form of powder as outlined in Table 4.

Before pressing, the gum base granules with the tablet base material in the form of powder, the gum base granules passed a standard horizontal vibration sieve for removing any particles larger than 1.3 mm.

In one alternative, the first powdered tablet base material was subsequently conveyed to a rotary tablet pressing machine (Hata tablet press) comprising dosing apparatus. The samples CG103 to CG106 were produced individually. About 0.5 g of the first powdered tablet base material was added and slightly pressed into a first pressed material of about 0.5 g. The force in this pre-pressing step was about 10 kN. Thereafter, a capsule (0.2 g of gelatine with a nutraceutical ingredient, chitosan) having an outer membrane was inserted by a device into a preformed cavity made by the upper punch of the tablet press. This object was fixed in the cavity made by the upper punch and was not forced out of the cavity during rotation. The object was about 30% below the face of the pre-pressed tablet base material. Thereafter a second powdered portion of tablet base (Table 4) was added (0.5 g) to the punch die fully covering the capsule in the punch die. The main pressing was performed with a force about 33.0 - 33.6 kN. The tablet was ejected.

In another alternative, the first powdered tablet base material was subsequently conveyed to a rotary tablet pressing machine (Hata tablet press) comprising dosing apparatus. The samples CG103 to CG106 were produced individually. About 0.5 g of the first powdered tablet base material was added and slightly pressed into a first pressed material of about 0.5 g. The force in this pre-pressing step was about 10 kN. Thereafter, a capsule (0.2 g of polyol syrup) having an outer membrane was inserted by a device into a preformed cavity made by the upper punch of the tablet press. This object was fixed in the cavity made by the upper punch and was not forced out of the cavity during rotation. The object was about 30% below the face of the pre-pressed tablet base material. Thereafter a second powdered portion of tablet base (Table 4) was added (0.5 g) to the punch die fully covering the capsule in the punch die. The main pressing was performed with a force about 33.0 - 33.6 kN. The tablet was ejected.

In another alternative, the first powdered tablet base material was subsequently conveyed to a rotary tablet pressing machine (Hata tablet press) comprising dosing apparatus. The samples CG103 to CG106 were produced individually. About 0.5 g of the first powdered tablet base material was added and slightly pressed into a first pressed material of about 0.5 g. The force in this pre-pressing step was about 10 kN. Thereafter, a capsule (0.1 g of polyol syrup) having an outer membrane was inserted by a device into a preformed cavity made by the upper punch of the tablet press. This object was fixed in the cavity made by the upper punch and was not forced out of the cavity during rotation. The object was about 30% below the face of the pre-pressed tablet base material. Thereafter a second powdered portion of tablet base (Table 4) was added (0.5 g) to the punch die fully covering the capsule in the punch die. The main pressing was performed with a force about 33.0 - 33.6 kN. The tablet was ejected.

In another alternative, the first powdered tablet base material was subsequently conveyed to a rotary tablet pressing machine (Hata tablet press) comprising dosing apparatus. The samples CG103 to CG106 were produced individually. About 0.5 g of the first powdered tablet base material was added and slightly pressed into a first pressed material of about 0.5 g. The force in this pre-pressing step was about 10 kN. Thereafter, a capsule (0.2 g of polyol syrup) having an outer membrane was inserted by a device into a preformed cavity made by the upper punch of the tablet press. This object was fixed in the cavity made by the upper punch and was not forced out of the cavity during rotation. The object was about 50% below the face of the pre-pressed tablet base material. Thereafter a second powdered portion of tablet base (Table 4) was added (0.5 g) to the punch die fully covering the capsule in the punch die. The main pressing was performed with a force about 33.0 - 33.6 kN. The tablet was ejected.

In another alternative, the first powdered tablet base material was subsequently conveyed to a rotary tablet pressing machine (Hata tablet press) comprising dosing apparatus. The samples CG103 to CG106 were produced individually. About 1.0 g of the first powdered tablet base material was added and slightly pressed into a first pressed material of about 1.0 g. The force in this pre-pressing step was about 10 kN. Thereafter, a capsule (0.4 g of polyol syrup) having an outer membrane was inserted by a device into a preformed cavity made by the upper punch of the tablet press. This object was fixed in the cavity made by the upper punch and was not forced out of the cavity during rotation. The object was about 50% below the face of the pre-pressed tablet base material. Thereafter a second powdered portion of tablet base (Table 4) was added (1.0 g) to the punch die fully covering the capsule in the punch die. The main pressing was performed with a force about 33.0 - 33.6 kN. The tablet was ejected.

In another alternative, the first powdered tablet base material was subsequently conveyed to a rotary tablet pressing machine (Hata tablet press) comprising dosing apparatus. The samples CG103 to CG106 were produced individually. About 1.0 g of the first powdered tablet base material was added and slightly pressed into a first pressed material of about 1.0 g. The force in this pre-pressing step was about 0.01 kN. Thereafter, a capsule (0.4 g of polyol syrup) having an outer membrane was inserted by a device into a preformed cavity made by the upper punch of the tablet press. This object was fixed in the cavity made by the upper punch and was not forced out of the cavity during rotation. The object was about 50% below the face of the pre-pressed tablet base material. Thereafter a second powdered portion of tablet base (Table 4) was added (1.0 g) to the punch die fully covering the capsule in the punch die. The main pressing was performed with a force about 33.0 - 33.6 kN. The tablet was ejected.

In another alternative, the first powdered tablet base material was subsequently conveyed to a rotary tablet pressing machine (Hata tablet press) comprising dosing apparatus. The samples CG103 to CG106 were produced individually. About 1.0 g of the first powdered tablet base material was added and slightly pressed into a first pressed material of about 1.0 g. The force in this pre-pressing step was about 0.01 kN. Thereafter, a capsule (0.4 g of polyol syrup) having an outer membrane was inserted by a device into a preformed cavity made by the upper punch of the tablet press. This object was fixed in the cavity made by the upper punch and was not forced out of the cavity during rotation. A further fixing syrup was added in the bottom of the cavity. The object was about 50% below the face of the pre-pressed tablet base material. Thereafter a second powdered portion of tablet base (Table 4) was added (1.0 g) to the punch die fully covering the capsule in the punch die. The main pressing was performed with a force about 33.0 - 33.6 kN. The tablet was ejected.

In another alternative, the first powdered tablet base material was subsequently conveyed to a rotary tablet pressing machine (Hata tablet press) comprising dosing apparatus. The samples CG103 to CG106 were produced individually. About 1.0 g of the first powdered tablet base material was added and slightly pressed into a first pressed material of about 1.0 g. The force in this pre-pressing step was about 0.01 kN. Thereafter, a capsule (0.4 g of polyol syrup) having an outer membrane was inserted by a device directly into the pre-pressed tablet base material. This object was fixed in the cavity by itself and was not forced out of the cavity during rotation. The object was about 50% below the face of the pre-pressed tablet base material. Thereafter a second powdered portion of tablet base (Table 4) was added (1.0 g) to the punch die fully covering the capsule in the punch die. The main pressing was performed with a force about 33.0 - 33.6 kN. The tablet was ejected.

In another alternative, the first powdered tablet base material was subsequently conveyed to a rotary tablet pressing machine (Hata tablet press) comprising dosing apparatus. The samples CG103 to CG106 were produced individually. About 1.0 g of the first powdered tablet base material was added and slightly pressed into a first pressed material of about 1.0 g. The force in this pre-pressing step was about 0.01 kN. Thereafter, a capsule (0.4 g of gelatine with a functional ingredient) having an outer membrane was inserted by a device directly into the pre-pressed tablet base material. This object was fixed in the cavity by itself and was not forced out of the cavity during rotation. The object was about 50% below the face of the pre-pressed tablet base material. Thereafter a second powdered portion of tablet base (Table 4) was added (1.0 g) to the punch die fully covering the capsule in the punch die. The main pressing was performed with a force about 33.0 - 33.6 kN. The tablet was ejected.

In another alternative, the first powdered tablet base material was subsequently conveyed to a rotary tablet pressing machine (Hata tablet press) comprising dosing apparatus. The samples CG103 to CG106 were produced individually. About 1.0 g of the first powdered tablet base material was added and slightly pressed into a first pressed material of about 1.0 g. The force in this pre-pressing step was about 0.01 kN. Thereafter, a capsule (0.4 g of gelatine with a functional ingredient) having an outer membrane was inserted by a device directly into the pre-pressed tablet base material. This object was fixed in the cavity by itself and was not forced out of the cavity during rotation. The object was about 50% below the face of the pre-pressed tablet base material. Thereafter a second powdered portion of tablet base (Table 4) was added (1.0 g) to the punch die fully covering the capsule in the punch die. The main pressing was performed with a force about 20 kN. The tablet was ejected.

In another alternative, the first powdered tablet base material was subsequently conveyed to a rotary tablet pressing machine (Hata tablet press) comprising dosing apparatus. The samples CG103 to CG106 were produced individually, but in this alternative without GB. About 0.5 g of the first powdered tablet base material was added and slightly pressed into a first pressed material of about 0.5 g. The force in this pre-pressing step was about 10 kN. Thereafter, a capsule (0.2 g of polyol syrup) having an outer membrane was inserted by a device into a preformed cavity made by the upper punch of the tablet press. This object was fixed in the cavity made by the upper punch and was not forced out of the cavity during rotation. The object was about 50% below the face of the pre-pressed tablet base material. Thereafter a second powdered portion of tablet base (Table 4) was added (0.5 g) to the punch die fully covering the capsule in the punch die. The main pressing was performed with a force about 33.0 - 33.6 kN. The tablet was ejected.

In another alternative, the first powdered tablet base material was subsequently conveyed to a rotary tablet pressing machine (Hata tablet press) comprising dosing apparatus. The samples CG103 to CG106 were produced individually, but in this alternative without GB. About 0.5 g of the first powdered tablet base material was added and slightly pressed into a first pressed material of about 0.5 g. The force in this pre-pressing step was about 10 kN. Thereafter, a capsule (0.2 g of polyol syrup) having an outer membrane was inserted by a device into a preformed cavity made by the upper punch of the tablet press. This object was fixed in the cavity made by the upper punch and was not forced out of the cavity during rotation. The object was about 50% below the face of the pre-pressed tablet base material. Thereafter a second powdered portion of tablet base, but in this case without gum base (Table 4) was added (0.5 g) to the punch die fully covering the capsule in the punch die. The main pressing was performed with a force about 33.0 - 33.6 kN. The tablet was ejected.

In another alternative, the first powdered tablet base material was subsequently conveyed to a rotary tablet pressing machine (Hata tablet press) comprising dosing apparatus. The samples CG103 to CG106 were produced individually, but in this alternative without GB. About 0.5 g of the first powdered tablet base material was added and slightly pressed into a first pressed material of about 0.5 g. The force in this pre-pressing step was about 5 kN. Thereafter, a capsule (0.2 g of polyol syrup) having an outer membrane was inserted by a device into a preformed cavity made by the upper punch of the tablet press. This object was fixed in the cavity made by the upper punch and was not forced out of the cavity during rotation. The object was about 50% below the face of the pre-pressed tablet base material. Thereafter a second powdered portion of tablet base, but in this case without gum base (Table 4) was added (0.5 g) to the punch die fully covering the capsule in the punch die. The main pressing was performed with a force about 20 kN. The tablet was ejected.

In another alternative, the first powdered tablet base material was subsequently conveyed to a rotary tablet pressing machine (Hata tablet press) comprising dosing apparatus. The samples CG103 to CG106 were produced individually, but in this alternative without GB. About 0.5 g of the first powdered tablet base material was added and slightly pressed into a first pressed material of about 0.5 g. The force in this pre-pressing step was about 1 kN. Thereafter, a capsule (0.2 g of gum base) having an outer membrane was inserted by a device into a preformed cavity made by the upper punch of the tablet press. This object was fixed in the cavity made by the upper punch and was not forced out of the cavity during rotation. The object was about 50% below the face of the pre-pressed tablet base material. Thereafter a second powdered portion of tablet base, but in this case without gum base (Table 4) was added (0.5 g) to the punch die fully covering the capsule in the punch die. The main pressing was performed with a force about 20 kN. The tablet was ejected.

## Claims

1. Method of producing a compressed tablet with a liquid or semi-liquid centre in a rotary tablet press, the method comprising the steps of
i) adding a first powdered portion of tablet base material into a punch die of a rotary tablet press,
ii) pressing said first tablet base material by means of an upper and a lower punch to obtain a first pressed material,
iii) inserting an object at the upper face of the first pressed material and fixing the object in a central cavity of the first pressed material,
iv) adding a second powdered portion of tablet base material into the punch die of the rotary tablet press, and
v) pressing said first and second tablet base material around the object to enclose the object from the surface of the tablet,
wherein the object is based on a material different from the first and/or the second powdered portion of tablet base material,
wherein the object comprises a liquid and/or a semi-liquid and a membrane as a barrier,
wherein the weight ratio between the powdered tablet base material in the tablet and the object is 20:1 to 3:1, and
wherein at least one of the first and the second powdered portion of tablet base material comprises granules of gum base or chewing gum.

2. Method according to claim 1, wherein the first powdered portion and/or the second powdered portion of tablet base material comprises particles of polyol sweetener.

3. Method according to any of the preceding claims, wherein the first powdered portion of tablet base material comprises granules of chewing gum and/or granules of gum base.

4. Method according to any of the preceding claims, wherein the amount of granules of gum base is in the range of 5 to 80% by weight of the first powdered portion of tablet base material, such as in the range of 10 to 70% by weight, such as in the range of 20 to 60% by weight, such as in the range of 30 to 50% by weight.

5. Method according to any of the preceding claims, wherein the second powdered portion of tablet base material comprises granules of chewing gum and/or granules of gum base.

6. Method according to any of the preceding claims, wherein the amount of granules of gum base is in the range of 5 to 80% by weight of the second powdered portion of tablet base material, such as in the range of 10 to 70% by weight, such as in the range of 20 to 60% by weight, such as in the range of 30 to 50% by weight.

7. Method according to any of the preceding claims, wherein the first powdered portion of tablet base material or the second powdered portion of tablet base material does not comprise gum base.

8. Method according to any of the preceding claims, wherein the first powdered portion of tablet base material is different from the second powdered portion of tablet base material.

9. Method according to any of the preceding claims, wherein the object is situated in the cavity substantially in the centre of the tablet.

10. Method according to any of the preceding claims, wherein the surface of the object is subjected to equal force per square milimeter during pressing said first and second tablet base material around the object.

11. Method according to any of the preceding claims, wherein the object is substantially ball-shaped.

12. Method according to any of the preceding claims, wherein the object comprises gelatine and a membrane as a barrier.

13. Method according to any of the preceding claims, wherein the object comprises an active ingredient, such as a nutraceutical ingredient, a pharmaceutical active ingredient, a functional ingredient, or any combination thereof.

14. Method according to any of the preceding claims, wherein the weight ratio between the powdered tablet base material in the tablet and the object is 15:1 to 4:1.

## Patentansprüche

1. Verfahren zur Herstellung einer gepressten Tablette mit einer flüssigen oder halbflüssigen Mitte in einer Rundläufertablettenpresse, wobei das Verfahren die folgenden Schritte umfasst:
i) Hinzufügen eines ersten pulverförmigen Teils des Tablettenausgangsmaterials in eine Stempelmatrize einer Rundläufertablettenpresse,
ii) Pressen des ersten Tablettenausgangsmaterials durch einen oberen und einen unteren Stempel, um ein erstes gepresstes Material zu erhalten,
iii) Einführen eines Objekts auf der Oberseite des ersten gepressten Materials und Fixieren des Objekts in einer zentralen Kavität des ersten gepressten Materials,
iv) Hinzufügen eines zweiten Tablettenausgangsmaterials in die Stempelmatrize der Rundläufertablettenpresse, und
v) Pressen des ersten und zweiten Tablettenausgangsmaterials um das Objekt, um das Objekt von der Oberfläche der Tablette einzuschließen,
wobei das Objekt auf einem Material basiert, das sich von dem ersten und/oder dem zweiten pulverförmigen Teil des Tablettenausgangsmaterials unterscheidet,
wobei das Objekt eine Flüssigkeit und/oder eine Halbflüssigkeit und eine Membran als Sperre umfasst,
wobei das Gewichtsverhältnis zwischen dem pulverförmigen Tablettenausgangsmaterial in der Tablette und dem Objekt 20:1 bis 3:1 beträgt, und
wobei mindestens ein Teil des ersten und des zweiten pulverförmigen Teils des Tablettenausgangsmaterials Granulat aus Gummibasis oder Kaugummi umfasst.

2. Verfahren nach Anspruch 1, wobei der erste pulverförmige Teil und/oder der zweite pulverförmige Teil des Tablettenausgangsmaterials Teilchen eines Polyol-Süßstoffes umfasst.

3. Verfahren nach einem der vorstehenden Ansprüche, wobei der erste pulverförmige Teil des Tablettenausgangsmaterials Granulat aus Kaugummi und/oder Granulat aus Gummibasis umfasst.

4. Verfahren nach einem der vorstehenden Ansprüche, wobei die Menge des Granulats aus Gummibasis im Bereich von 5 bis 80 Gewichtsprozent des ersten pulverförmigen Teils des Tablettenausgangsmaterials liegt, wie etwa im Bereich von 10 bis 70 Gewichtsprozent, wie etwa im Bereich von 20 bis 60 Gewichtsprozent, wie etwa im Bereich von 30 bis 50 Gewichtsprozent.

5. Verfahren nach einem der vorstehenden Ansprüche, wobei der zweite pulverförmige Teil des Tablettenausgangsmaterials Granulat aus Kaugummi und/oder Granulat aus Gummibasis umfasst.

6. Verfahren nach einem der vorstehenden Ansprüche, wobei die Menge des Granulats aus Gummibasis im Bereich von 5 bis 80 Gewichtsprozent des zweiten pulverförmigen Teils des Tablettenausgangsmaterials liegt, wie etwa im Bereich von 10 bis 70 Gewichtsprozent, wie etwa im Bereich von 20 bis 60 Gewichtsprozent, wie etwa im Bereich von 30 bis 50 Gewichtsprozent.

7. Verfahren nach einem der vorstehenden Ansprüche, wobei der erste pulverförmige Teil des Tablettenausgangsmaterials oder der zweite pulverförmige Teil des Tablettenausgangsmaterials keine Gummibasis umfasst.

8. Verfahren nach einem der vorstehenden Ansprüche, wobei sich der erste pulverförmige Teil des Tablettenausgangsmaterials von dem zweiten pulverförmigen Teil des Tablettenausgangsmaterials unterscheidet.

9. Verfahren nach einem der vorstehenden Ansprüche, wobei sich das Objekt in der Kavität im Wesentlichen in der Mitte der Tablette befindet.

10. Verfahren nach einem der vorstehenden Ansprüche, wobei die Oberfläche des Objekts während dem Pressen des ersten und zweiten Tablettenausgangsmaterials um das Objekt der gleichen Kraft pro Quadratmillimeter ausgesetzt ist.

11. Verfahren nach einem der vorstehenden Ansprüche, wobei das Objekt im Wesentlichen kugelförmig ist.

12. Verfahren nach einem der vorstehenden Ansprüche, wobei das Objekt Gelatine und eine Membran als Sperre umfasst.

13. Verfahren nach einem der vorstehenden Ansprüche, wobei das Objekt einen aktiven Inhaltsstoff umfasst, wie etwa einen nutrazeutischen Inhaltsstoff, einen Arzneistoff, einen funktionellen Inhaltsstoff oder eine Kombination dieser.

14. Verfahren nach einem der vorstehenden Ansprüche, wobei das Gewichtsverhältnis zwischen dem pulverförmigen Tablettenausgangsmaterial in der Tablette und dem Objekt von 15:1 bis 4:1 beträgt.

## Revendications

1. Procédé de production d'un comprimé compressé muni d'un centre liquide ou semi-liquide dans une presse à comprimés rotative, le procédé comprenant les étapes consistant à :
i) ajouter une première portion en poudre du matériau de base de comprimé dans une matrice de poinçonnage d'une presse à comprimés rotative,
ii) presser ledit premier matériau de base de comprimé au moyen d'un poinçon supérieur et d'un poinçon inférieur afin d'obtenir un premier matériau pressé,
iii) insérer un objet au niveau de la face supérieure du premier matériau pressé et fixer l'objet dans une cavité centrale du premier matériau pressé,
iv) ajouter une deuxième portion en poudre de matériau de base de comprimé dans la matrice de poinçonnage de la presse à comprimés rotative, et
v) presser ledit premier et deuxième matériau de base de comprimé autour de l'objet afin d'entourer l'objet à partir de la surface du comprimé,
l'objet étant à base d'un matériau différent de la première et/ou de la deuxième portion en poudre de matériau de base de comprimé,
l'objet comprenant un liquide et/ou un semi-liquide et une membrane en tant que barrière,
le rapport en poids entre le matériau de base de comprimé en poudre dans le comprimé et l'objet étant de 20:1 à 3:1, et
au moins un élément parmi la première et la deuxième portion en poudre de matériau de base de comprimé comprenant des granulés de gomme de base ou de gomme à mâcher.

2. Procédé selon la revendication 1, la première portion en poudre et/ou la deuxième portion en poudre de matériau de base de comprimé comprenant des particules d'édulcorant polyol.

3. Procédé selon l'une quelconque des revendications précédentes, la première portion en poudre de matériau de base de comprimé comprenant des granulés de gomme à mâcher et/ou des granulés de gomme de base.

4. Procédé selon l'une quelconque des revendications précédentes, la quantité de granulés de gomme de base étant dans la plage allant de 5 à 80 % en poids de la première portion en poudre de matériau de base de comprimé, tel que dans la plage allant de 10 à 70 % en poids, tel que dans la plage allant de 20 à 60 % en poids, tel que dans la plage allant de 30 à 50 % en poids.

5. Procédé selon l'une quelconque des revendications précédentes, la deuxième portion en poudre de matériau de base de comprimé comprenant des granulés de gomme à mâcher et/ou des granulés de gomme de base.

6. Procédé selon l'une quelconque des revendications précédentes, la quantité de granulés de gomme de base étant dans la plage allant de 5 à 80 % en poids de la deuxième portion en poudre de matériau de base de comprimé, tel que dans la plage allant de 10 à 70 % en poids, tel que dans la plage allant de 20 à 60 % en poids, tel que dans la plage allant de 30 à 50 % en poids.

7. Procédé selon l'une quelconque des revendications précédentes, la première portion en poudre de matériau de base de comprimé ou la deuxième portion en poudre de matériau de base de comprimé ne comprenant pas de gomme de base.

8. Procédé selon l'une quelconque des revendications précédentes, la première portion en poudre de matériau de base de comprimé étant différente de la deuxième portion en poudre de matériau de base de comprimé.

9. Procédé selon l'une quelconque des revendications précédentes, l'objet étant situé dans la cavité essentiellement au centre du comprimé.

10. Procédé selon l'une quelconque des revendications précédentes, la surface de l'objet étant soumise à une force égale par millimètre carré pendant le pressage dudit premier er deuxième matériau de base de comprimé autour de l'objet.

11. Procédé selon l'une quelconque des revendications précédentes, l'objet étant essentiellement en forme de bille.

12. Procédé selon l'une quelconque des revendications précédentes, l'objet comprenant de la gélatine et une membrane en tant que barrière.

13. Procédé selon l'une quelconque des revendications précédentes, l'objet comprenant un ingrédient actif, tel qu'un ingrédient nutraceutique, un ingrédient actif pharmaceutique, un ingrédient fonctionnel ou une combinaison quelconque de ceux-ci.

14. Procédé selon l'une quelconque des revendications précédentes, le rapport en poids entre le matériau de base de comprimé en poudre dans le comprimé et l'objet étant de 15:1 à 4:1.
